# FASCICULE DE BREVET EUROPEEN

(11) **EP 1 194 136 B1**
(45) Date de publication et mention de la délivrance du brevet: **18.02.2004**
(21) Numéro de dépôt: 99923685.4
(22) Date de dépôt: 08.06.1999
(51) Int. Cl.: A61K 31/045, A61K 47/10, A61K 47/26, A61K 47/00, A61P 17/02, A61K 35/78

(54) **COMPOSITION NON SOLIDE POUR APPLICATION LOCALE COMPRENANT DU GLYCEROL ET UN EXTRAIT D'ALCHEMILLE VULGAIRE**
NICHT-FESTES TOPISCH ANZUWENDENDES ARZNEIMITTEL ENTHALTEND GLYZEROL UND ALCHEMILLA VULGARIS EXTRAKT
NON-SOLID COMPOSITION FOR LOCAL APPLICATION COMPRISING GLYCEROL AND ALCHEMILLA VULGARIS EXTRACT

(43) Date de publication de la demande: 10.04.2002
(73) Titulaire: NATURVEDA, 43250 Sainte Florine (FR)
(72) Inventeur: Shrivastava, Ravi, Dr., 63118 Cebazat (FR)
(74) Mandataire: Vidon, Patrice
(86) Numéro de dépôt international: PCT/FR1999/001340
(87) Numéro de publication internationale: WO 2000/074668

(56) Documents cités:
- EP-A- 0 579 155
- WO-A-98/03152
- FR-A- 2 332 026
- FR-A- 2 773 077
- DATABASE WPI Derwent Publications Ltd., London, GB; AN 1984-144200 [23] XP002130212 & RO 82 747 A (INTR. MEDICAMENTE BIOFARM.) 30 octobre 1983

## Description

La présente invention conceme de nouvelles compositions non solides notamment pharmaceutiques contenant une solution hypertonique de glycérol et, un extrait d'Alchémille vulgaire et leur utilisation pour le traitement des aphtes et des blessures superficielles.

Le développement des lésions sous la forme d'aphtes au niveau de la bouche, et occasionnellement sur d'autres parties du corps est une affection très fréquente. La plupart des individus sont susceptibles de développer des aphtes au niveau buccal et d'être sujet à de petites blessures. Bien que les aphtes ne constituent pas une maladie à part entière, ils sont à l'origine de douleurs importantes et d'inconforts significatifs.

Du point de vue de la physiopathologie, un aphte peut être considéré comme étant une rupture localisée de l'intégrité des zones superficielles de la peau ou des muqueuses. Cette blessure expose les tissus sous-jacents et plus profonds à des traumatismes plus étendus, qui se manifestent par la rupture des vaisseaux et l'endommagement des parties plus profondes que la membrane muqueuse ou de l'épiderme. Ces petites plaies sont exposées aux micro-organismes, et en particulier aux bactéries responsables d'infections secondaires, comme les streptocoques et les staphylocoques, micro-organismes qui provoquent secondairement des lésions plus profondes sous la forme d'ulcères buccaux.

Le développement de telles lésions au niveau de la peau s'associe à des blessures traumatiques et des démangeaisons, mais la formation des aphtes au niveau des muqueuses peut également être liée à d'autres facteurs qui ne sont pas actuellement complètement connus. En plus des lésions traumatiques, l'apparition des aphtes et des ulcères buccaux peut également être due à certains éléments de l'alimentation qui provoquent l'altération des muqueuses. La déficience en certaines vitamines, comme la vitamine A, est une cause de fragilité de la muqueuse qui se rompt facilement suite à de petites blessures.
Cliniquement, les aphtes ou les blessures superficielles sont des petites lésions des muqueuses ou de l'épiderme (de quelques millimètres à quelques centimètres), violacées ou jaunâtres, qui laissent apparaître les couches sous-jacentes des tissus et les vaisseaux capillaires sanguins. Ces lésions constituent un site idéal pour la prolifération bactérienne. La présence de bactéries pyrogènes est un phénomène commun. Le système de défense et les processus de régénération de l'organisme sont immédiatement activés après la formation des blessures et commencent le processus de guérison. Le système immunitaire lutte contre la prolifération bactérienne et infectieuse afin de-préparer la zone endommagée aux phénomènes de régénération.

Bien que ces processus soient relativement rapides pour les blessures de la peau, cela prend au moins sept à dix jours pour la guérison complète des aphtes buccaux. Cette durée importante de rémission est liée au fait que les aphtes sont en contact constant avec les aliments qui contiennent des germes non pathogènes. Ainsi, le lieu atteint est constamment exposé aux micro-organismes prêts à se multiplier dans un environnement favorable. Les mouvements constants à l'intérieur de la bouche, par exemple lors de la parole, augmentent également le temps nécessaire à la régénération et retardent la réparation des blessures.

Les traitements actuellement disponibles consistent à stopper ou à réduire la multiplication bactérienne au niveau de la blessure sans favoriser la régénération tissulaire préalable à la guérison. La plupart des traitements disponibles pour les aphtes contiennent des antibiotiques ou des agents antiseptiques. Ces traitements consistent en des applications locales. Dans le cas d'infections sévères, une antibiothérapie par voie orale est utilisée. Un inconvénient majeur de ces traitements est qu'ils agissent uniquement sur les infections bactériennes secondaires de la blessure mais ne possèdent pas d'activité directe sur la régénération tissulaire.

Bien souvent, le sujet atteint a tendance à gratter la zone atteinte, ce qui provoque une inflammation et peut aggraver l'étendue des lésions. Ainsi, un autre inconvénient non négligeable des traitements actuellement disponibles est qu'ils ne réduisent pas le temps de rémission des sujets atteints qui continuent à ressentir des douleurs très inconfortables et peuvent aggraver les blessures déjà importantes.
Ces traitements possèdent également des effets secondaires, communs aux antibiothérapies.

Ainsi, un traitement idéal des aphtes devrait posséder trois qualités principales :
- Eliminer les micro-organismes présents au niveau de la lésion, afin de créer un terrain favorable à la multiplication cellulaire.
- Accélérer la régénération tissulaire pour accélérer le rétablissement et réduire au maximum le temps de rémission.
- Etre non toxique et ne pas être responsable d'effets secondaires.

A ce jour, aucun produit ne possédant à la fois ces trois propriétés d'élimination des bactéries de la blessure, et de cicatrisation en plus de l'absence de toxicité, n'a été découvert.

La glycérine ou les solutions concentrées, par exemple les solutions concentrées de sucres ont souvent été utilisées comme conservateur, comme dans les confitures, ou encore comme excipients, mais une quelconque activité pharmacologique, notamment pour le traitement des aphtes ou des blessures superficielles, n'a jamais été décrite pour ces produits.

Or la demanderesse a découvert avec étonnement que les bactéries peuvent être éliminées de la blessure ouverte en un temps très court par l'application d'une solution concentrée, osmotiquement active vis à vis du plasma, et qu'en outre le temps nécessaire pour obtenir la guérison était considérablement réduit grâce à l'utilisation d'une substance qui augmente la multiplication cellulaire.

C'est pourquoi la présente demande a pour objet une composition non solide et de préférence liquide pour application locale qui comprend, au moins du glycérol ou une solution concentrée de glycérol et un extrait d'Alchémille vulgaire la concentration en principe actif de ladite composition non solide étant osmotiquement active vis à vis du plasma, notamment du plasma sanguin.

Dans des conditions préférentielles de mise en oeuvre de l'invention la composition non solide est une composition pharmaceutique.

Dans la présente demande et dans ce qui suit, le terme « non solide » appliqué à une composition selon l'invention vise tant les préparations liquides que semi- liquides (visqueuses).

D'après les observations de la demanderesse, du glycérol pur ou une solution concentrée de saccharose, de sorbitol, de mannitol ou de glycérine (glycérol), appliquée sur une blessure superficielle ouverte provoque un phénomène de drainage et d'accélération de la guérison. Ce mécanisme de drainage est le résultat de phénomènes osmotiques, de réactions inflammatoires et humorales (vasodilatation), immunitaires (migration de cellules immunocompétentes au niveau du site atteint), qui ne sont pas connus en détail. Sous l'influence des lois de la diffusion, le glycérol ou la solution hypertonique aurait tendance à pénétrer dans les tissus. Cependant, cette pénétration est limitée par la taille des molécules qui ne peuvent atteindre la circulation sanguine. Par contre, le plasma présent au niveau des capillaires endommagés qui présentent une perméabilité augmentée, est exsudé vers la blessure afin de rétablir l'équilibre osmotique. Ainsi, l'application d'une solution concentrée, hypertonique, au niveau de la blessure entraîne l'exsudation d'une grande quantité de plasma. Au cours de ce processus, les micro-organismes présents au niveau de la blessure sont éliminés avec le flux de plasma, ce qui a pour effet de réduire rapidement la charge bactérienne. Ainsi, les solutions concentrées permettent un drainage des petites plaies comme les aphtes et les blessures superficielles.

Cette exsudation de plasma apporte également de nombreux facteurs immunitaires (immunoglobulines, système du complément, leucocytes...) qui participent également à l'élimination des bactéries, ce qui crée un terrain favorable pour la guérison de la blessure.

Par ailleurs le glycérol ou les solutions concentrées de saccharose, de sorbitol, de mannitol, de glycérine (glycérol) sont très peu toxiques, voire inoffensifs pour la santé et sont utilisables par voie orale, sans induire aucun effet secondaire.

Dans des conditions préférentielles de mise en oeuvre de l'invention, on utilise à titre de principe actif dans la composition non solide le glycérol associé à un extrait d'Alchémille vulgaire. On préfère aussi utiliser dans la composition non solide de l'invention le sorbitol, ou le mannitol en association avec un extrait d'Alchémille vulgaire.

Dans d'autres conditions préférentielles de mise en oeuvre de l'invention, la concentration en principe actif dans la composition non solide doit permettre d'obtenir une solution au pouvoir osmotique supérieur à celui du plasma, à savoir supérieur à 300 milliosmoles (mOsm), de préférence supérieur à 500 mOsm, notamment supérieur à 800 mOsm, et tout particulièrement supérieur à 1 Osm. Il est bien entendu que ce pouvoir osmotique est conféré par ledit principe actif pour au moins 30 %, de préférence au moins 60 %, notamment au moins 80 %, particulièrement au moins 90 %, et tout particulièrement au moins 95 %, le reste du pouvoir osmotique pouvant être apporté par d'autres composés osmotiquement actifs.

Dans encore d'autres conditions préférentielles de mise en oeuvre de l'invention, la concentration en principe actif dans la composition non solide est telle que la quantité du diluant (solvant) en volume est inférieure à 70 %, de préférence inférieure à 40 %, notamment inférieure à 20 %, et tout particulièrement inférieure à 10 %.

Le mélange des substances osmotiquement actives ci-dessus avec des produits antibiotiques ou antiseptiques d'origine synthétique ou naturelle permet d'augmenter l'effet antibactérien. Le mélange des substances osmotiquement actives ci-dessus avec un composé possédant des propriétés de stimulation de la prolifération cellulaire, augmente également la vitesse de guérison de la blessure.

Pour ces raisons la présente demande a encore pour objet une composition non solide telle que définie dans la revendication 1 ci-dessus dans laquelle au moins une substance osmotiquement active est associée avec au moins un produit antiseptique ou un produit stimulant la multiplication cellulaire. Une telle association présente une solution efficace pour le traitement des aphtes, des blessures superficielles, des brûlures, et les soins postopératoires afin de stimuler la guérison en réduisant les cicatrices.

Ainsi, on peut en particulier trouver dans les compositions non solides une ou plusieurs substances capables de stimuler la prolifération cellulaire, et notamment des extraits de plantes utilisées traditionnellement ou non dans le traitement des affections dermatologiques (Mimosa tenuiflora, Quercus, Aesculus hippocastanum, Geranium robertianum, Cupressus sempervirens, Vitis vinifera, Ribes nigrum, Centella asiatica, Matricaria Chamomilla et tout particulièrement Alchemilla Vulgaris), ou tout autre substance dotée d'une activité type « facteur de croissance » (exemples : escine, tanins-oligomères procyanidoliques, mimosides), ou encore au moins un antibiotique, bactériostatique ou bactéricide (exemple : papaïne, géraniine).

Ces compositions notamment pharmaceutiques peuvent être, par exemple, liquides ou semi-liquides et se présenter sous les formes pharmaceutiques couramment utilisées en médecine humaine, comme par exemple les solutions en tubes avec embout allongé ou en pulvérisateur ; elles sont préparées selon les méthodes usuelles. Le ou les principes actifs peuvent y être incorporés à des excipients habituellement employés dans les compositions pharmaceutiques, tels que les véhicules aqueux ou non, les divers agents mouillants, les conservateurs, les épaississants.

L'invention a aussi pour objet du glycérol ou une solution concentrée de. glycérol, de saccharose, de sorbitol ou de mannitol, en concentration osmotiquement active vis à vis du plasma, en association avec un extrait d'Alchémille vulgaire pour leur utilisation dans une méthode de traitement thérapeutique du corps humain ou animal, c'est-à-dire à titre de médicament.

Les médicaments selon la présente invention trouvent leur emploi par exemple dans le traitement tant curatif que préventif des aphtes. Ils trouvent aussi leur emploi dans le traitement des ulcères des muqueuses ou de l'épiderme autres que les aphtes.

La dose usuelle, variable selon le sujet traité et l'affection en cause, peut être, par exemple, de 2 applications à 6 applications par jour par voie locale chez l'homme de 2 gouttes d'une composition de l'exemple 3 sur chaque aphte, pendant 3 jours.

La présente invention a encore pour objet un procédé de préparation d'une composition ci-dessus décrite, caractérisé en ce que l'on mélange, selon des méthodes connues en elles mêmes, le ou les principes actifs avec des excipients ou solvants acceptables, notamment pharmaceutiquement acceptables.

L'invention a enfin pour objet l'utilisation du glycérol ou une solution concentrée de glycérol, de saccharose, de sorbitol du de mannitol, en concentration osmotiquement active vis à vis du plasma, pour l'obtention d'un médicament destiné au traitement des petites lésions des muqueuses ou de l'épiderme et notamment des aphtes.

Les conditions préférentielles de mise en oeuvre des compositions non solides et de préférence liquides ci-dessus décrites s'appliquent également aux autres objets de l'invention visés ci-dessus.

Les exemples qui suivent illustrent la présente demande.

### EXEMPLE 1 (exemple de référence)

On a préparé des tubes de 10 ml avec embout de 4 cm répondant à la formule

| | |
|---|---|
| Eau | 60 ml |
| Sorbitol | 40 g |

Agiter jusqu'à dissolution complète.

### EXEMPLE 2 (exemple de référence)

On a préparé des tubes de 10 ml avec embout de 4 cm répondant à la formule

| | |
|---|---|
| Eau | 50 ml |
| glycérol | 50 ml |

### EXEMPLE 3

On a préparé des tubes de 10 ml avec embout de 4 cm répondant à la formule pondérale :

| | |
|---|---|
| eau | 45% |
| gomme xanthane | 0,5% |
| para hydroxy benzoate de méthyle | 0,15% |
| extrait hydroalcoolique d'Alchémille * | 5% |
| arome cassis | 0,43% |
| glycérol | q.s.p. 100% |

| | |
|---|---|
| * Origine BIOSPHERE - FRANCE : 150 g de feuilles sèches dans 500 ml d'eau et 500 ml d'éthanol. | |

### EXEMPLE 4

On a préparé des tubes de 10 ml avec embout de 4 cm répondant à la formule:

| | |
|---|---|
| Glycérine | 97 ml |
| Extrait sec d'Alchemilla vulgaris | 3 g |

Mélanger.

### EXEMPLE 5 (exemple de référence)

On a préparé des tubes de 10 ml avec embout de 4 cm répondant à la formule :

| | |
|---|---|
| Glycérol | 90 % |
| Extrait de Cassis | 9 % |
| Extrait d'Azadirachta indica | 1 % |

Mélanger.

### EXEMPLE 6 :

On a préparé des tubes de 10 ml avec embout de 4 cm répondant à la formule :

| | |
|---|---|
| Glycérine | 96,5 % |
| Extrait type d'Alchemilla vulgaris | 3 % |
| Extrait d'Azadirachta indica | 0.5% |

### EXEMPLE 7 : (exemple de référence)

On a préparé des tubes de différentes contenances répondant à la formule:

| | |
|---|---|
| Extrait de marron d'Inde | 8.1 % |
| Extrait de Cyprès | 5.0% |
| Extrait de Géranium Robert | 4.0% |
| Escine | 0.3% |
| Papaïne | 0.1% |
| Carbomer | 0.5% |
| Alcool | 4.0% |
| Phénonip | 0.5% |
| PEG-7 Gylcéryl cocoate | 3.0% |
| Glycérol | 30% |
| Eau q.s.p. | 100% |

### EXEMPLE 8 :

On a préparé des tubes de différentes contenances répondant à la formule:

| | |
|---|---|
| Alchémille vulgaire | 9.8% |
| Vigne rouge | 2.0% |
| Mimosa tenuiflora | 5.0% |
| Carbomer | 0.4% |
| PEG-7 Gylcéryl cocoate | 2.0% |
| Phénonip | 0.5% |
| Triethanolamine | 0.2% |
| Fragance | 0.2% |
| Glycérol | 10-40% |
| Eau q.s.p. | 100% |

### EXEMPLE 9: (exemple de référence)

On a préparé des tubes de différentes contenances répondant à la formule:

| | |
|---|---|
| Extrait de Quercus | 0.5% |
| Escine | 0.1% |
| Azadirachta indica | 1.1 % |
| Méthyl parahydroxybenzoate | 0.15% |
| Gomme xanthane | 0.5% |
| Extrait de Cassis | 0.43% |
| Glycérol | 50% |
| Eau q.s.p. | 100% |

### ETUDE PHARMACOLOGIQUE

### Effet de la glycérine associée à un extrait de plante utilisée traditionnellement dans le traitement des affections dermatologiques

30 rats (IOPS IFFA-CREDO de 200 +/-20 g) ont été rasés (3 x 3 cm) sur le flanc droit du dos. Une blessure de 0,4 x 0,4 cm a été pratiquée au milieu de la zone rasée. 30 minutes après avoir réalisé la petite blessure, le sang coagulé a été éliminé et 0,2 ml de glycérine contenant 3 % d'Alchemilla vulgaris (Préparation de l'exemple 4) ou 0,2 ml de glycérine pure (témoins) ont été appliqués sur dix animaux. Les dix autres rats témoin ont reçu 0.2 ml d'eau distillée. Le temps de guérison complète et l'index de cicatrisation ont été déterminés chaque jour pendant 10 jours. Le temps de cicatrisation a été diminué de 48 % avec le traitement à base de glycérine contenant 3 % d'Alchémilla vulgaris, avec un index de cicatrisation de 2,1 dans le lot traité, par rapport à un index de 3,3 obtenu avec le lot témoin.

Avec la glycérine seule, le temps de cicatrisation a été diminué de 26 % avec un index de cicatrisation de 2,7. Ces résultats indiquent que la glycérine seule facilite la cicatrisation des blessures, mais également qu'une association avec un produit capable de stimuler fortement les mitoses cellulaires augmente la vitesse de cicatrisation.

Les effets de différents extraits de plantes sur la vitesse de prolifération des cellules épithéliales ont été déterminés in vitro. Les cellules de reins de bovins (MDBK) ont été cultivées en plaques de 96 puits (10⁵ cellules / ml ; 100 µl par puit).

Différentes concentrations d'extraits de plantes ont été ajoutées dans le milieu de culture pendant la préparation des cellules (n=16 par concentration). Les cellules ont été incubées pendant 72 heures (37°C ; 5 % CO₂) et le nombre total de cellules a été déterminé après trypsination et coloration au MTT. Seuls 8 extraits de plantes sur 26 étudiés stimulent la mitose à plus de 12 % dans l'ordre suivant : Alchemilla vulgaris, Mimosa tenuiflora, Quercus, Aesculus hippocastanum, Geranium robertianum, Cupressus sempervirens, Vitis vinifera, Ribes nigrum.

### ETUDE CLINIQUE

On a mis en tubes de 10 ml, d'une part, une préparation contenant 97 % de glycérine et 3 % d'un extrait hydroglycériné d'Alchemilla vulgaris (à 3 % d'extrait sec de plante p/p), selon l'exemple 4, et d'autre part, une préparation contenant 97 % d'alcool éthylique à 96 % et 3 % d'un extrait hydroglycolique d'Alchemilla vulgaris (à 3 % d'extrait sec de plante p/p).

18 sujets ayant régulièrement des problèmes d'aphtes buccaux ont été inclus dans une étude clinique pilote 8 sujets témoins ont testé l'extrait hydroalcoolique tandis que 10 sujets ont testé l'extrait hydroglycériné. Deux gouttes de produit ont été appliquées 3 fois par jour après les repas jusqu'à la guérison complète. Le temps nécessaire pour obtenir une rémission complète a été déterminé avec les deux groupes.
Le temps moyen de guérison est de 2,7 jours dans le lot traité avec l'extrait hydroglycériné par rapport à 6,3 jours avec le groupe témoin.

L'utilisation de substances osmotiquement actives ou de la glycérine, seules ou en association avec d'autres composés stimulant les mitoses cellulaires, améliore les phénomènes de prolifération cellulaire, de cicatrisation des blessures superficielles et notamment de guérison des aphtes.

## Revendications

1. Composition non solide pour application locale pour le traitement des aphtes et des blessures cutanées **caractérisée ce qu**'elle comprend du glycérol et/ou du saccharose et/ou du sorbitol et/ou du mannitol et un extrait d'Alchémille vulgaire et en ce qu'elle présente un pouvoir osmotique supérieur à celui du plasma sanguin, c'est-à-dire supérieur à 300 milliosmoles.

2. Composition non solide pour application locale selon la revendication 1, **caractérisée en ce qu'**elle comprend du glycérol et un extrait d'Alchémille vulgaire.

3. Composition non solide pour application locale selon la revendication 1, **caractérisée en ce qu'**elle comprend du sorbitol ou du mannitol et un extrait d'Alchémille vulgaire.

4. Composition non solide pour application locale selon l'une quelconque des revendications 1 à 3 **caractérisée en ce qu'**elle présente un pouvoir osmotique supérieur à 500 milliosmoles.

5. Composition non solide pour application locale selon la revendication 4 **caractérisée en ce qu'**elle présente un pouvoir osmotique supérieur à 800 milliosmoles.

6. Composition non solide pour application locale selon la revendication **caractérisée en ce** en ce qu'elle présente un pouvoir osmotique supérieur à 1 osmole.

7. Composition non solide pour application locale selon l'une quelconque des revendications 1 à 6 **caractérisée en ce qu'**elle comprend une quantité de diluant (solvant) représentant moins de 20% du volume de la composition.

8. Composition non solide pour application locale selon l'une des revendications 1 à 7 **caractérisée en ce qu'**elle comprend de plus un antibiotique et/ou un antiseptique et/ou un produit stimulant la multiplication cellulaire.

9. Utilisation du glycérol et/ou du saccharose et/ou du mannitol et/ou du sorbitol en concentration osmotiquement active vis à vis du plasma sanguin en association avec un extrait d'Alchémille vulgaire, pour l'obtention d'une composition destinée au traitement des ulcères buccaux et des affections cutanées.

## Patentansprüche

1. Nicht-feste Zusammensetzung zur topischen Anwendung für die Behandlung von Aphten und Hautverletzungen, **dadurch gekennzeichnet, dass** sie Glycerin und/oder Saccharose und/oder Sorbitol und/oder Mannitol und einen Extrakt aus *Alchemilla vulgaris* umfasst, und dass sie einen osmotischen Druck größer als den von Blutplasma besitzt, nämlich größer als 300 mosm.

2. Nicht-feste Zusammensetzung zur topischen Anwendung gemäß Anspruch 1, **dadurch gekennzeichnet, dass** sie Glycerin und einen Extrakt aus *Alchemilla vulgaris* enthält.

3. Nicht-feste Zusammensetzung zur topischen Anwendung nach Anspruch 1, **dadurch gekennzeichnet, dass** sie Sorbitol oder Mannitol und einen Extrakt aus *Alchemilla vulgaris* enthält.

4. Nicht-feste Zusammensetzung zur topischen Anwendung nach einem der Ansprüche 1 bis 3, **dadurch gekennzeichnet, dass** sie einen osmotischen Druck größer als 500 mosm besitzt.

5. Nicht-feste Zusammensetzung zur topischen Anwendung nach Anspruch 4, **dadurch gekennzeichnet, dass** sie einen osmotischen Druck größer als 800 mosm besitzt.

6. Nicht-feste Zusammensetzung zur topischen Anwendung nach Anspruch 5, **dadurch gekennzeichnet, dass** sie einen osmotischen Druck größer als 1 osm besitzt.

7. Nicht-feste Zusammensetzung zur topischen Anwendung nach einem der Ansprüche 1 bis 6, **dadurch gekennzeichnet, dass** sie einen Anteil an Verdünnungsmittel (Lösungsmittel) von weniger als 20 % bezogen auf das Volumen der Zusammensetzung umfasst.

8. Nicht-feste Zusammensetzung zur topischen Anwendung nach einem der Ansprüche 1 bis 7, **dadurch gekennzeichnet, dass** sie zusätzlich ein Antibiotikum und/oder ein Antiseptikum und/oder ein die Zellvermehrung stimulierendes Produkt umfasst.

9. Verwendung von Glycerin und/oder Saccharose und/oder Mannitol und/oder Sorbitol in einer gegenüber Blutplasma osmotisch aktiven Konzentration in Verbindung mit einem Extrakt von *Alchemilla vulgaris* zum Herstellen einer Zusammensetzung für die Behandlung von Geschwüren des Mundes und von Hauterkrankungen.

## Claims

1. A non-solid composition for local application for the treatment of mouth ulcers and cutaneous injuries **characterised in that** it comprises glycerol and/or sucrose and/or sorbitol and/or mannitol and an extract of Alchemilla vulgaris and **in that** it has an osmotic strength greater than that of blood plasma, namely greater than 300 milliosmoles.

2. Non-solid composition for local application according to claim 1, **characterised in that** it comprises glycerol and an extract of Alchemilla vulgaris.

3. Non-solid composition for local application according to claim 1, **characterised in that** it comprises sorbitol or mannitol and an extract of Alchemilla vulgaris.

4. Non-solid composition for local application according to any one or claims 1 to 3, **characterised in that** it has an osmotic strength greater than 500 milliosmoles.

5. Non-solid composition for local application according to claim 4 **characterised in that** it has an osmotic strength greater than 800 milliosmoles.

6. Non-solid composition for local application according to claim 5 **characterised in that** it has an osmotic strength greater than 1 osmole.

7. Non-solid composition for local application according to any one of claims 1 to 6, **characterised in that** it includes a quantity of diluent (solvent) representing less than 20 % of the volume of the composition.

8. Non-solid composition for local application according to one of claims 1 to 7 **characterised in that** it additionally includes an antibiotic and/or an antiseptic and/or a product stimulating cell multiplication.

9. Use of glycerol and/or sucrose and/or mannitol and/or sorbitol in an osmotically active concentration in relation to blood plasma in association with an extract of Alchemilla vulgaris, in order to obtain a composition intended for the treatment of mouth ulcers and cutaneous diseases.
